# EUROPEAN PATENT SPECIFICATION

(11) **EP 1 346 026 B1**
(45) Date of publication and mention of the grant of the patent: **01.04.2009**
(21) Application number: 01272379.7
(22) Date of filing: 26.12.2001
(51) Int. Cl.: C12P 19/32, C12R 1/15

(54) **MICROORGANISM PRODUCING 5'-INOSINIC ACID AND PROCESS FOR PRODUCING 5'-INOSINIC ACID USING THE SAME**
5'-INOSINSÄURE HERSTELLENDER MIKROORGANISMUS UND VERFAHREN ZUR HERSTELLUNG VON 5'- INOSINSÄURE UNTER VERWENDUNG DESSELBEN
MICROORGANISME PRODUISANT DE L'ACIDE 5'-INOSINIQUE ET PROCEDE SERVANT A PRODUIRE L'ACIDE 5'-INOSINIQUE AU MOYEN DE CE MICROORGANISME

(30) Priority: 26.12.2000 KR 2000081471
(43) Date of publication of application: 24.09.2003
(73) Proprietor: CJ Cheiljedang Corporation, Seoul 100-749 (KR)
(72) Inventor: KIM, Hyun-Soo, Dongan-ku, Anyang, Kyunggi-do 431-050 (KR); CHUNG, Sung-Oh, Sungnam, Kyunggi-do 463-747 (KR); LEE, Jin-Ho, Suji-up, Yongin, Kyunggi-do 449-846 (KR); KANG, Sung-Goo, Songpa-ku, Seoul 138-808 (KR); KIM, Jeong-Hwan, Seoul 138-812 (KR); HWANG, Soo-Youn, Yongin, Kyunggi-do 449-846 (KR); LEE, Byung-Chon, Seoul 136-075 (KR); LEE, Jae-Chul, Kyunggi-do 467-814 (KR)
(74) Representative: Grünecker, Kinkeldey, Stockmair & Schwanhäusser Anwaltssozietät
(86) International application number: PCT/KR2001/002264
(87) International publication number: WO 2002/051984

(56) References cited:
- EP-A- 0 816 491
- JP-A- 2 312 595
- JP-A- 5 007 439
- JP-A- 11 009 295
- TOMITA, KAZUHIRO ET AL: "Stimulation by L-proline of 5'- inosinic acid production by mutants of Corynebacterium ammoniagenes" AGRICULTURAL AND BIOLOGICAL CHEMISTRY, vol. 55, no. 9, 1991, pages 2221-2225, XP002234343
- REITZER, L. J. AND MAGASANIK, B.: "20. Ammonia Assimilation and the Biosynthesis of Glutamine, Glutamate, Aspartate, Asparagine, L-Alanine, and D-Alanine" ESCHERICHIA COLI AND SALMONELLA TYPHIMURIUM CELLULAR AND MOLECULAR BIOLOGY, 1987, pages 302-320, XP001247677 AMERICAN SOCIETY FOR MICROBIOLOGY, WASHINGTON, DC, US
- NEUHARD, J. AND NYGAARD, P.: "29. Purines and Pyrimidines" ESCHERICHIA COLI AND SALMONELLA TYPHIMURIUM CELLULAR AND MOLECULAR BIOLOGY, 1987, pages 445-473, XP001247663 AMERICAN SOCIETY FOR MICROBIOLOGY, WASHINGTON, DC, US

## Description

### TECHNICAL FIELD

The present invention relates to a novel microorganism producing 5-inosinic acid and to a process for producing 5'-inosinic acid using the same.

### BACKGROUND ART

5'-inosinic acid is an intermediate material of the metabolic system of nucleic acid biosynthesis, which is used in a variety of fields, like foods and medicines, and in various kinds of medical areas and has an important meaning in the animal and plant physiologically, and, in particular, is one of the nucleic acid-type seasonings which are spotlighted as a taste seasonings having the synergic effect when used with sodium glutamate.

The processes for producing 5'-inosinic acid by a direct fermentation have been known in this field, and the important key in economical aspects was to produce 5'-inosinic acid in a high concentration and yield.

Tomita K et al. (1991, Agricultural and Biological Chemistry 55(9):2221-2225) discloses the effect of L-proline on 5'-inosinic acid production from a mutant strain of *Corynebacterium ammoniagenesis,* KY10895.

EP-A-0 816 491 discloses a protein having inosine-guanosine kinase activity. D2 further discloses a method for producing 5'-inosinic acid or 5'-guanylic acid employing a microorganism capable of reproducing ATP and transformed with a gene encoding the inosine-guanosine kinase.

JP-A-2 312 595 discloses mutant strains of *Corynebacterium Ammoniagenes* and their use in the production of 5'-inosinic acid.

### DISCLOSURE OF THE INVENTION

The present inventors conducted extensive studies to develop a new strain capable of achieving the above mentioned purposes, and as a result, discovered a novel microorganism producing 5'-inosinic acid by a direct fermentation in a high concentration and yield.

The present invention provides a *Corynebacterium ammoniagenes* CJIP009 (KCCM-10226) characterized by accumulating 5'-inosinic acid in a high concentration and yield by a direct fermentation and having resistance to L-glutamine analogues selected from Azaserine or 6-diazo-5-oxo-L-norleucine (DON), and resistance to L-proline analogues selected from 3,4-dehydroproline, L-azetidine-2-c-arboxylic acid, L-thiamlidine-4-carboxylic acid, (S)-2,2-dimethyl-4-oxatolidecarboxylic acid, (S)-5,5-dimethyl-4-thiazolide carboxylic acid, (4S,2RS)-2-ethyl-4-thiazofino-carboxylic acid, (2S,4S)-4-hydroxy-2-pyrroline-carboxylic acid, 2-piperidinecarboxylic acid or 2,5-pyrrolidinedione.

The present invention also provides a process for producing 5'-inosinic acid characterized by cultivating a *Corynebacterium ammoniagenes* CJIP009 (KCCM-(0226) followed by collection of the cultivated substances.

According to the present invention, a microorganism, a mutant of *Corynebacterium ammoniagenes* (ATCC-6872), requires Adenine, but does not require Xanthine or Guanine, though the growth is facilitated by adding them, when compared with a conventional Adenine Leaky Mutant producing 5'-inosinic acid [Agr, Bio, Chem., Vol 47(5), p1035∼1041, 1983, (KY13102, KY13171, KY13184, etc.)], or the microorganism simutaneously requiring Adenine and Xanthine or Guanine.

In addition, the microorganism of the present invention lacks of Urease to assimilate Urea, and has a high sensitivity to lysozyme, cell wall degradation enzyme, which is considered that capacity of cell wall synthesis is partially lost so that lots of intracellularly produced 5'-inosinic acid is easily secreted out of the cell.

Balabushevich, M.I. and Kazarinova, L.A., et al (Prikl. Biokhim. Mikrobiol., 19(5), 590∼598, 1983), Russia, have discovered that adding streptomycin and kanamycin to the medium enhances the permeability and helps accumulation of 5'-inosinic acid in the medium. Considering this, it was contemplated that a mutant, capable of producing 5'-inosinic acid in a high concentration and yield, would have been discovered by introducing resistance of streptomycin to a known strain to enhance the membrane permeability of the microorganism. Then, it was contemplated that the contamination frequently occurring in fermentation would be prevented by using the above mutant and adding the streptomycin to the medium. Therefore, the present inventors obtained a strain having resistance to a high concentration of streptomycin and studied properties of the strain. In practice, it was identified that introduction of streptomycin-resistance of a high concentration to the strain effectively prevents contamination occurring during fermentation.

Most bacteria accumulate calcium ion and organic solutes, i.e., osmolytes, by the method of improving intracellular osmotic pressure of bacteria to prevent osmotic dehydration under extracellular osmotic pressure of bacteria. As such osmolytes, there have been known L-proline, L-glutamate, sugar, N-methylated amino acid derivatives, etc. Among them, L-proline has been known as an important factor of osmoregulation and *Brevibacterium typhimurium* reported [Agr, Bio, Chem., Vol 53(9), p2475-2479, 1989] that L-proline was accumulated intracellularly, on increasing the activity of pyrroline-5-carboxylate reductase, which is the important enzyme of biosynthesis pathway of proline, under the osmotic condition by extracellular increased 5'-inosinic acid. Besides, it was reported [J, Bacteriol., Vol 163, p296, 1985] that L-proline was accumulated intracellularly in *Escherichia coli, Salmonella typhimurium, Serratia marcescens,* etc. and regulated by extracellular osmotic pressure.

Accordingly, it is contemplated that for a microorganism capable of producing 5'-inosinic acid in a high concentration and yield, it is important to prevent the inhibition of growth and biochemical metabolic process by increasing intracellular L-proline synthesis and that it is important to reinforce the osmotic pressure-resistant characteristics by enhancing the synthetic capacity of L-proline.

Further, in order to produce 5'-inosinic acid from 5-phosphoribosyl-α-1-pyrophosphate (PRPP), i.e., a precusor material of purine-type nucleic acid, 2 molecule of glutamine is needed. Glutamine synthetase, which is the enzyme producing glutamine from sodium glutamate, is very elaborately regulated by amino acids, such as glycine, alanine, histidine, etc., and CTP, AMP, etc. [Reitzer LJ and Magasanik B, Escherichia coli and Salmonella typhimurium, 1987, p362∼320]. Therefore, a harmonious supply of the glutamine is necessary for the synthesis of 5'-inosinic acid. Further, produced glutamine is widely used as a precursor of various reactions. In the synthesis of 5'-inosinic acid, two enzymes, which are PRPP amidotransferase and 5-phosphoribosyl-N-formylglycinamide (FGAR) amidotransferase, employ glutamine as a substrate [Neuhard J and Nygaard P, Escherichia coli and Salmonella typhimurium, 1987, p445∼473]. Therefore, in order to more effectively achieve 5'-inosinic acid synthesis, it is considered that the harmonious synthesis of the glutamine is important to increase the affinity of PRPP amidotransferase and FGAR amidotransferase related to synthesis of 5'-inosinic acid to the glutamine rather than other enzymes among various reaction requiring glutamine.

Accordingly, the present inventors tested how various amino acids would affect the synthesis of 5'-inosinic acid by a direct fermentation. As the result, when L-glutamine was added to the medium of 5'-inosinic acid, the inventors identified that fermentation concentration of 5'-inosinic acid is increased and that the appropriate supply of L-glutamine to the present strain is a rate-limiting step in the synthesis of 5'-inosinic acid.

The inventors identified that the microorganisms introduced resistance to L-glutamine analogues, such as Azaserine or 6-diazo-5-oxo-L-norleucine (DON), and resistance to various L-proline analogues, such as 3,4-dehydroprotine, L-azeddine-2-carboxylic acid, L-thiazolidine-4-carboxylic acid, (S)-2,2-dimethyl-4-oxazolidecarboxylic acid, (S)-5,5-dimethyl-4-thiazolide carboxylic acid, (4S,2RS)-2-ethyl-4-thiazoline-carboxylic acid, (2S,4S)-4-hydroxy-2-pyrroline-2carboxylic acid, 2-piperidinecarboxylic acid or 2,5-pyrrolidinedione can produce 5'-inosinic acid by a direct fermentation method in a higher concentration and yield than the known strains. The present invention is based on such discovery.

Hereunder, the present invention will be more specifically explained.

First, the microorganism of the present invention, a mutant of *Corynebacterium ammoniagenes* (ATCC-6872), requires Adenine, but does not require Xanthine or Guanine, though the growth is facilitated by adding them, and the microorganism lacks of Urease to assimilate Urea, and has a high sensitivity to lysozyme, cell wall degradation enzyme, which is considered that capacity of cell wall synthesis is partially lost, so that lots of intracellularly produced 5'-inosinic acid is easily secreted out of the cell, and has resistance to streptomycin in order to effectively correspond to a contamination occurring during fermentation.

High concentration of glucose, other carbon sources added during culture period, and 5'-inosinic acid accumulated during the later culture period lead to an increase in extracellular osmotic pressure of 5'-inosinic acid-producing microorganisms thereby inhibiting their normal physiological activities and cell growth. Therefore, it is desirable to improve the osmotic resistance to prevent the reduction of 5'-inosinic acid production.

To increase the intracellular concentration of proline which plays an important role in osmoregulation to a high accumulation of solutes in the extracellular environment, the microorganism of the present invention has resistance to L-proline analogues, such as 3,4-dehydroproline, L-azetidine-2-carboxylic acid, L-thiazolidine-4-carboxylic acid, (S)-2,2-dimethyl-4-oxazolidecarboxylic acid, (S)-5,5-dimethyl-4-thiazolide carboxylic acid, (4S,2RS)-2-ethyl-4-thiazoline-carboxylic acid, (2S,4S)-4-hydroxy-2-pyrroline-carboxylic acid, 2-piperidinecarboxylic acid or 2,5-pyrrolinedione, thereby excluding the effect of osmotic pressure more efficiently.

Further, the microorganism of the present invention has resistance to L-glutamine analogues, such as Azaserine or 6-diazo-5-oxo-L-norleucine (DON), essentially required in the purine- type synthesis system by achieving the harmonious supply of glutamine for the synthesis of 5'-inosinic acid, resulting in directly accumulating 5'-inosinic acid in a high yield and concentration.

The mutant of the present invention obtained a colony from the microorganism (CJ112), which requires Adenine, but does not require Xanthine or Guanine, though growth is facilitated by adding them, and lacks of Urease to assimilate Urea, as a parent strain, by being treated with X-ray, ultraviolet ray, and a chemical organic mutagen such as, N-methyl-N'-nitro-N-nitrosoguanidine (NTG), diethyl sulfate, ethyl amine, etc., and by being suitably suspended and spread on a minimal medium (Medium 2) containing 1.7% agar and each concentration of variants. Then, each colony was cultivated on a nutrition medium (Medium 1), and then cultivated on a seed medium (Medium 3) for 24 hours, and then cultivated on a fermentation medium (Medium 4) for S∼6 days thereby to select a microorganism that can produce 5'-inosinic acid accumulated in the culture medium at the largest amounts, step by step. Then, the present inventors selected a strain that can produce 5'-inosinic acid accumulated in the culture medium at the largest amounts among the microorganisms in the final step. The above strain was designated as CJIP009. *Corynebacterium ammoniagenes* CJIP009 was deposited under the Budapest Treaty to the Korean Culture Center of Microorganisms whose address is Hongje-dong, Seodaemun-gu, Seoul, on November 20, 2000, with the Accession No. KCCM-10226.

specifically, the present invention provides a process for producing 5'-inosinic acid characterized in that a mutant of *Corynebacterium ammoniagenes* CJlP009 (KCCM-10226) according to Claim 1 is cultivated on a seed medium at 30 °C for 24 hours, cultivated and activated on a fermentor seed medium at 28-34 °C, 900rpm and pH 7.2 for 1-2 days, cultivated on a fermentor main medium at 30 °C, 900rpm and pH 7.2 for 5-6 days, and then when a reducing sugar is 2% in the culture solution, the first, second, third and fourth additional sugars are mixed with fructose, glucose and molasses and the respective final sugars are added and cultivated to be 32%.

Culture media employed in the present invention have the following compositions:
Medium 1: Nutrition medium
   peptone 1%, Beef extract 1%, Sodium Chloride (NaCl) 0.25%, Yeast Extract 1%, Agar 2%, pH 7. 2
Medium 2: Minimal medium
   Glucose 2.0%, Ammonium Sulfate ((NH₄)₂SO₄) 0.3%, Potassium Dihydrogen Phosphate (KH₂PO₄) 0.1%, Potassium Monohydrogen Phosphate (K₂HPO₄) 0.3%, Magnesium Sulfate (MgSO₄·7H₂O) 0.3%, Calcium chloride (CaCl₂) 10 mg/l, Ferric Sulfate FeSO₄·7H₂O) 10 mg/l, Zinc Sulfate (ZnSO₄·7H₂O) 1.0 mg/l, Manganese Chloride (MnCl₂-H₂O) 3.6 mg/l, L-Cystein 20 mg/l,, Calcium Pantothenate 10 mg/l, Thiamine HCl 5.0 mg/l, Biotin 30 µg/l, Adenine 20 mg/l, Guanine 20 mg/l, pH 7.3
Medium 3: Seed medium
   Glucose 5%, peptone 0.5%, Beef extract 0.5%, Yeast Extract 1%, Sodium Chloride (NaCl) 0.25%, Adenine 100 mg/l, Guanine 100 mg/l, pH 7.2
Medium 4: Flask fermentation medium
   Sodium Glutamate 0.1%, Ammonium Chloride (NH₄Cl) 1.0%, Magnesium Sulfate (MgSO₄ 7H₂O) 1.2%, Calcium Chloride (CaCl₂ 0.01%, Ferric Sulfate (FeSO₄·7H2O) 20 mg/l, Manganese Sulfate (N_{b}SO₄-H₂O) 20 mg/l, Zinc Sulfate (ZnSO₄·7H₂O) 20 mg/l, Cupric Sulfate (CuSO₄-7H₂O) 5.0 mg/l, L-Cystein 23 mg/l, Alanine 24 mg/l, Nicotinic acid 8.0 mg/l, Biotin 45 µg/l, Thiamine-HCl 5.0 mg/l. Adenine 30 mg/l, phosphoric acid (H₃PO₄)(85%) 1.9%, the mixture of Fructose, Glucose and molasses to 8% as reducing sugar (pH 7.2)
Medium 5: Fermentator seed medium
   Glucose 5.4%, peptone 1.0%, Yeast Extract 2.0%, Potassium Dihydrogen Phosphate (KH₂PO₄) 0.1%, Potassium Monohydrogen Phosphate (K₂HPO₄) 0.1%, Magnesium Sulfate (MgSO₄·7H₂O) 0.1%, Ammonium Sulfate ((NH₄)₂SO₄) 0.5%, Ferric Sulfate (FeSO₄·7H₂O) 80 mg/l, Zinc Sulfate (ZnSO₄·7H₂O) 40 mg/l, Manganese Sulfate (MnSO₄·H₂O) 40 mg/l, L-Cystein 80 mg/l,, Calcium Pantothenate 60 mg/l, Thiamine·HCl 20 mg/l, Biotin 240 µg/l, Adenine 1200 mg/l, Guanine 1200 mg/l (pH 7.2)
Medium 6: Fermentor main medium
   Calcium Chloride (CaCl₂ 120 mg/l, Cupric Sulfate (CUSO₄·7H₂O) 8.0 mg/l, Magnesium Sulfate (MgSO₄·7H₂O) 1.5%, Ferric Sulfate (FeSO₄·7H₂O) 24 mg/l, Zinc Sulfate (ZnSO₄·7H₂O) 24 mg/l, Manganese Sulfate (MnSO₄·H₂O) 24 mg/l, L-Cystein 26.4 mg/l, Sodium Glutamate 0.12%, Thiamine·HCl 6.0 mg/l, Biotin 40 µg/l, Nicotinic acid 50 mg/l, Alanine 145 mg/l, Adenine 200 mg/l, phosphoric acid (H₃PO₄)(85%) 4.3%, the mixture of Fructose, Glucose and molasses to 32% as reducing sugar (pH 7.2)

The biochemical properties of representative variants of a novel mutant CJIP009 according to the present invention is shown in the following Table 1 (This invention is not limited to the following properties).

**Table 1**

| Property | ATCC6872 | CJIP009 (KCCM-10226) |
|---|---|---|
| Adenine | Non-require | Require |
| Guanine (Xanthine) | Non-require | Leaky |
| Sensitivity of lysozyme (minimal growth inhibition concentration) | 80µg/ml | 8µg/ml |
| Resistance to 3,4-dehydroproline | 1,000µg/ml | 3,500µg/ml |
| Streptomycin | 500µg/ml | 2,000µg/ml |
| L-azetidine-2-carboxylic acid | 5mg/ml | 30 mg/ml |
| L-thiazolidine-4-carboxylic acid | 10µg/ml | 100µg/ml |
| Azaserine | 25µg/ml | 100µg/ml |

The culture process of 5'-inosinic acid used in the invention was as follows.

The microorganisms, belonging to *Corynebacterium* genus and capable of producing 5'-inosinic acid, were cultured in the conventional medium containing carbon sources, nitrogen sources, amino acids, vitamins, etc., under aerobic condition with regulated temperature, pH, etc.

Glucose, fructose, sterilized pre-treated molasses (molasses reverted to reducing sugar) and so on, would be used as a carbon source. Among inorganic nitrogen sources such as, ammonia, ammonium chloride, ammonium sulfate and organic nitrogen sources such as, peptone, NZ-amine, meat extract, yeast extract, corn digestive solution, casein hydrolysate, fishes or degradation products thereof, de-fatted soybean cake or degradation products thereof and so on, each would be used as a organic nitrogen source. Potassium Dihydrogen Phosphate (KH₂PO₄), Potassium Monohydrogen Phosphate (K₂HPO₄), Manganese Sulfate (MnSO4·H₂O), Ferric Sulfate (FeSO₄·7H₂O), Magnesium Sulfate (MgSO₄·7H₂O), Calcium Carbonate (CaCO₃), etc., would be used as the inorganic compounds. If required, vitamins and base, etc., would be added. The culture is performed for example, while shaking or aerating and agitating under aerobic condition, preferably at 20∼40°C for 5-6 days. The pH of the medium preferably remains around neutrality. The 5'-inosinic acid accumulated by a direct fermentation is analyzed by the conventional method.

### BEST MODE FOR CARRYING OUT THE INVENTION

This invention will be better understood from the following examples. However, one skilled in the art will readily appreciate the specific materials and the results described below are merely illustrative of, and are not intended to, nor should be intended to, limit the invention as described more fully in the claims which follows thereafter.

### Example 1: Selection of microorganism sensitive to lysozyme (LY002)

The mutant of the example 1 was prepared from the microorganism (CJ112) from the *Brevibacterium ammoniagenes* (ATCC-6872), which requires Adenine, but does not require Xanthine or Guanine, though growth is facilitated by adding them and lacks of Urease to assimilate Urea as the parent strain. The strain was suspended to 10⁷∼10⁸ cells/ml in the phosphate buffer (pH 7.0) or citrate buffer (pH 5.5), and induced mutation by adding N-methyl-N'-nitro-N-nitrosoguanidine (NTG) to the final concentration of 10-50µg/ml at room temperature or 32°C for 20∼40 minutes, and washed twice with 0.85% saline. The colonies were obtained being suitably suspended and spread on a minimal medium (Medium 2) containing 1.7% agar. Then, each colony was tooth picked on the minimal medium (Medium 2) containing 1.7% agar and on the minimal medium (Medium 2) containing 1.7% agar and 40µg/ml of lysozyme. First, the microorganism, which was grown on the minimal medium containing 1.7% agar and not on the minimal medium containing 40µg/ml of lysozyme, was selected. The microorganism was to be a parent strain and to be continuously induced the above mutation. Second, the sensitive microorganism, which was not grown on the minimal medium containing 16µg/ml of lysozyme, was selected. Finally, according to the above method, the high sensitive microorganism which was not grown on the minimal medium containing 8µg/ml of lysozyme, was selected. The colony of the high sensitive microorganism was cultivated on a nutrition medium (Medium 1), then cultivated for 24 hours on a seed medium (Medium 3), and cultivated for 3∼4 days on a culture medium (Medium 4) thereby to select LY002 that can produce 5'-inosinic acid accumulated in the culture medium at the largest amounts. The concentration of lysozyme, at which the microorganism shows sensitivity, was listed in the following Table 2

**Table 2**

| | CJ112 | LY002 |
|---|---|---|
| Concentration of lysozyme | 80µg/ml | 8µg/ml |

### Example 2: Selection of streptomycin-resistant strain (CISM10)

In a mutant of the example 2, LY002 of Example 1 was used as a parent strain. The strain was suspended to 10⁷∼10⁸ cells/ml in the phosphate buffer (pH 7.0) or citrate buffer (pH 5.5) and induced mutation by adding NTG to the final concentration of 10∼50µg/ml at room temperature or 32°C for 20∼40 minutes, and washed twice with 0.85% saline. The colonies were obtained being suitably suspended and spread on 3 minimal media (Medium 2), each of which containing 1.7% agar and 1,000µg/ml, 1,500µg/ml or 2,000µg/ml of streptomycin, respectively. Then, each colony was cultivated on the nutrition medium (Medium 1), then cultivated for 24 hours on a seed medium (Medium 3), and cultivated for 3∼4 days on a culture medium (Medium 4) thereby to select CISM10 that can produce 5'-inosinic acid accumulated in the culture medium at the largest amounts. The concentration of streptomycin, at which the microorganism shows resistance, was listed in the following Table 3

**Table 3**

| | LY002 | CISM10 |
|---|---|---|
| Concentration of streptomycin | 500µg/ml | 2,000µg/ml |

### Example 3: Selection of 3,4-dehydroproline -resistant strain (CS101)

In a mutant of Example 3, CISM10 of Example 2 was used as a parent strain. The strain was suspended to 10⁷∼10⁸ cells/ml in the phosphate buffer (pH 7.0) or citrate buffer (pH 5.5) and induced mutation by adding NTG to the final concentration of 10-50µg/ml at room temperature or 32°C for 20∼40 minutes, and washed twice with 0.85% saline. The colonies were obtained by suitably suspend spread on 3 minimal media (Medium 2), each of which containing 1.7% agar and 1,50µg/ml, 2,500µg/ml, or 3,500µg/ml of 3,4-dehydroproline, respectively. Then, each colony was cultivated on the nutrition medium (Medium 1), then cultivated for 24 hours on a seed medium (Medium 3), and cultivated for 3∼4 days on a culture medium (Medium 4) thereby to select CIS104 that can produce 5'-inosinic acid accumulated in the culture medium at the largest amounts. The concentration of 3,4-dehydroproline, at which the microorganism shows resistance, was listed in the following Table 4

**Table 4**

| | CISM10 | CS101 |
|---|---|---|
| Concentration of 3,4-dehydroproline | 1,000µg/ml | 3,500µg/ml |

### Example 4: Selection ofL-azetidine-2-carboxylic acid-resistant strain (CIAC12)

In a mutant of Example 4, CS101 of Example 3 was to be a parent strain. The strain was suspended to 10⁷∼10⁸ cells/ml in the phosphate buffer (pH 7.0) or citrate buffer (pH 5.5) and induced mutation by adding NTG to the final concentration of 10∼50ug/ml at room temperature or 32°C, for 20-40 minutes, and washed twice with 0.85% saline. The colonies were obtained being suitably suspended and spread on 3 minimal media (Medium 2), each of which containing 1.7% agar and 10µg/ml, 20µg/ml, or 30µg/ml of L-azetidine-2-carboxylic acid, respectively. Then, each colony was cultivated on the nutrition medium (Medium 1), then cultivated for 24 hours on a seed medium (Medium 3), and cultivated for 3-4 days on a culture medium (Medium 4), thereby to select CIAC12 that can produce 5'-inosinic acid accumulated in the culture medium at the largest amounts. The concentration of L-azetidine-2-carboxylic acid, at which the microorganism shows resistance, was listed in the following Table 5.

**Table 5**

| Property | CISM10 | CIAC12 |
|---|---|---|
| Concentration of L-azetidine-2-carboxylic acid | 5mg/ml | 30mg/ml |

### Example 5: Selection ofL-thiazolidine-4-carboxylic acid-resistant strain (CITP13)

In a mutant of Example 5, CIAC12 of Example 4 was to be a parent strain. The strain was suspended to 10⁷∼10⁸ cells/ml in the phosphate buffer (pH 7.0) or citrate buffer (pH 5.5) and induced mutation by adding NTG to the final concentration of 10-50µg/ml at room temperature or 32°C for 20∼40 minutes, and washed twice with 0.85% saline. The colonies were obtained being suitably suspended and spread on 3 minimal media (Medium 2), each of which containing 1.7% agar and 20µg/ml, 50µg/ml, or 100µg/ml of L-thiazolidine-4-carboxylic acid, respectively. Then, each colony was cultivated on the nutrition medium (Medium 1), then cultivated for 24 hours on a seed medium (Medium 3), and cultivated for 3∼4 days on a culture medium (Medium 4) thereby to select CITP13 that can produce 5'-inosinic acid accumulated in the culture medium at the largest amounts. The concentration of L-thiazolidine-4-carboxylic acid, at which the microorganism show resistance, was listed in the following Table 6.

**Table 6**

| | CIAC12 | CITP13 |
|---|---|---|
| Concentration of L-thiazolidine-4-carboxylic acid | 10µg/ml | 100µg/ml |

### Example 6: Selection of azaserine -resistant strain CJIPP009 (KCCM-10226)

In a mutant of Example 6, CITP 13 of Example 5 was to be a parent strain. The strain was suspended to 10⁷∼10⁸ cells/ml in the phosphate buffer (pH 7.0) or citrate buffer (pH 5.5) and induced mutation by adding NTG to the final concentration of 10∼50µg/ml at room temperature or 32°C for 20∼40 minutes, and washed twice with 0.85% saline. The colonies were obtained being suitably suspended and spread on 3 minimal media (Medium 2), each of which containing 1.7% agar and 50µg/ml, 75µg/ml, or 100µg/ml of azaserine, respectively. Then, each colony was cultivated on the nutrition medium (Medium 1), then cultivated for 24 hours on a seed medium (Medium 3), and cultivated for 3∼4 days on a culture medium (Medium 4) thereby to select CJIP009 (KCCM-10226) that can produce 5'-inosinic acid accumulated in the culture medium at the largest amounts. The concentration of azaserine, at which the microorganism shows resistance, was listed in the following Table 7.

**Table 7**

| | CITP13 | CJIP009(KCCM-10226) |
|---|---|---|
| Concentration of azaserine | 25µg/ml | 100µg/ml |

### Example 7: Measurement of the accumulation amount of 5'-inosinic acid in Erlenmeyer flask

Strain:
   CJIP009 (KCCM-10226)
Seed medium:
   Glucose 5%, peptone 0.5%, Beef extract 0.5%, Yeast Extract 1%, Sodium Chloride (NaCl) 0.25%, Adenine 100 mg/l, Guanine 100 mg/l, pH 7.2
Flask fermentation medium
   Sodium Glutamate 0.1%, Ammonium Chloride (NH₄Cl) 1.0%, Magnesium Sulfate (MgSO₄·7H₂O) 1.2%, Calcium Chloride (CaCl₂) 0.01%, Ferric Sulfate (FeSO₄·7H₂O) 20 mg/l, Manganese Sulfate (MnSO₄·H₂O) 20 mg/l, Zinc Sulfate (ZnSO₄·7H_{z}O) 20 mg/l, Cupric Sulfate (CuSO₄·7H₂O) 5.0 mg/l, L-Cystein 23 mg/l, Alanine 24 mg/l Nicotinic acid 8.0 mg/l, Biotin 45 µ/gl, Thiamine·HCl 5.0 mg/l, Adenine 30 mg/l, phosphoric acid (H₃PO₄)(85%) 1.9%, the mixture of Fructose, Glucose and molasses to 8% as reducing sugar (pH 7.2)
Fermentation procedure:
   3ml of the seed medium was introduced into a test tube of 18mm diameter and sterilized under elevated pressure by the general method. Thereto was inoculated the strain, and cultivated while shaking at 30°C for 24 hours to use as the seed medium. ' 27ml of fermentation medium was introduced into 500ml of Erlenmeyer flask for shaking and sterilized under elevated pressure at 120°C for 10minutes. Thereto was inoculated 3ml of seed culture, and cultivated for 5∼6 days. The flask was shaked at 200rpm at the temperature of 30°C and pH of 7.2.
   The accumulated amount of 5'-inosinic acid was 19.1g/l.

### Example 8: Measurement of the accumulation amount of 5'-inosinic acid in 5L of fermentor

Strain:
   CJIP009 (KCCM-10226)
Seed medium: same as example 7
Fermentor seed medium
   Glucose 5.4%, peptone 1.0%, Yeast Extract 2.0%, Potassium Dihydrogen Phosphate (KH₂PO₄) 0.1%, Potassium Monohydrogen Phosphate (K₂HPO₄) 0.1%, Magnesium Sulfate (MgSO₄·7H₂O) 0.1%, Ammonium Sulfate (NH₄)₂SO₄) 0.5%, Ferric Sulfate (FeSO₄·7H₂O) 80 mg/l, Zinc Sulfate (ZnSO₄·7H₂O) 40 mg/l, Manganese Sulfate (MnSO₄·7H₂O) 40 mg/l, L-Cystein 80 mg/l,, Calcium Pantothenate 60 mg/l, Thiamine·HCl 20 mg/l, Biotin 240 µg/l, Adenine 1200 mg/l, Guanine.1200 mg/l (pH 7.2)
Fermentor main medium
   Calcium Chloride (CaCl₂ 120 mg/l, Cupric Sulfate (CuSO₄·7H₂O) 8.0 mg/l, Magnesium Sulfate (MgSO₄-7H₂O) 1.5%, Ferric Sulfate (FeSO₄·7H₂O) 24 mg/l, Zinc Sulfate (ZnSO₄·7H₂O) 24 mg/l, Manganese Sulfate (MnSO₄·7H₂O) 24 mg/l, L-Cystein 26.4 mg/l, Sodium Glutamate 0.12%, Thiamine·HCl 6.0 mg/l, Biotin 40 µg/l, Nicotinic acid 50 mg/l, Alanine 145 mg/l, Adenine 200 mg/l, phosphoric acid (H₃PO₄)(85%) 4.3%, the mixture of Fructose, Glucose and molasses to 32% as reducing sugar (pH 7.2)
Fermentation procedure:
   50ml of the seed medium was introduced into 500ml of Erlenmeyer flask for shaking and sterilized under elevated pressure by the general method. Thereto was inoculated the strain, and cultivated while shaking at 30 °C for 24 hours to use as the seed medium. 1,000ml of Fermentor seed medium was added to 2.5L of fermentor, and sterilized under elevated pressure 120 °C for 15 minutes. Thereto was inoculated 50ml of seed culture, and then cultivated for 1∼2 days. The flask was shaked at 900rpm at the temperature of 28∼34°C and pH of 7.2. Further, 1,250ml of Fermentor main medium was added to 5L of fermentor, and sterilized under elevated pressure at 120°C for 15 minutes. Thereto was inoculated 250ml of seed culture, and then cultivated for 1∼2 days. When reducing sugar in culture was 2% during cultivation, 1^{st}, 2^{nd}, 3^{rd}, and 4^{th} additional sugar, i.e., the mixture of fructose, glucose and molasses was added so that each sugar of the final reducing sugar became 32%. Then, the mixture was cultivated for 5∼6 days. The flask was shaked at 900rpm at the temperature of 30°C and pH of 7.2.
   The accumulated amount of 5'-inosinic acid in the medium was 70.3g/l.

### INDUSTRIAL APPLICABILITY

According to the present invention, 5'-inosinic acid can be obtained in a higher concentration and yield than prior art. According to the present invention, 5'-inosinic acid can be obtained more economically than prior art.

## Claims

1. A *Corynebacterium ammoniagenes* CJIP009 having accession number KCCM-10226, **characterized by** accumulating 5'-inosinic acid in a high concentration and yield by a direct fermentation and having resistance to L-glutamine analogues selected from Azaserine or 6-diazo-5-oxo-L-norleucine (DON), and resistance to L-proline analogues selected from 3,4-dehydroproline, L-azetidine-2-carboxylic acid, L-thiazolidine-4-carboxylic acid, (S)2,2-dimethyl-4-oxazolidecarboxylic acid, (S)-5,5-dimethyl-4-thiazoiide carboxylic acid, (4S, 2RS)-2-ethyl-4-thiazoline-carboxylic acid, (2S, 4S)-4-hydroxy-2-pyrroline-carboxylic acid, 2-piperidinecarboxylic acid or 2,5-pyrrolidinedione.

2. The *Corynebacterium ammoniagenes* as claimed in claim 1 **characterized in that** the *Corynebacterium ammoniagenes* is Guanine or Xanthine leaky type or the microorganism requiring Adenine.

3. The *Corynebacterium ammoniagenes* as claimed in claim 1 **characterized in that** the minimal growth inhibition concentration of lysozyme, cell wall degradation enzyme, is 8 µg/ml.

4. The *Corynebacterium ammoniagenes* as claimed in claim 1 **characterized by** having a resistance to streptomycin in order to prevent contamination occurring during fermentation.

5. A process for producing 5'-inosinic acid **characterized by** cultivating the *Corynebacterium ammoniagenes* according to Claim 1 followed by collection of the cultivated substances.

6. The process as claimed in claim 5 **characterized in that** the *Corynebacterium ammoniagenes* according to Claim 1 is cultivated on a seed medium at 30°C for 24 hours, cultivated and activated on a fermentor seed medium at 28-34°C, 900rpm and pH 7.2 for 1-2 days, cultivated on a fermentor main medium at 30°C, 900rpm and pH 7.2 for 5-6 days, and then when a reducing sugar is 2% in the culture solution, the first, second, third and fourth additional sugars are mixed with fructose, glucose and molasses and the respective final sugars are added and cultivated to be 32%.

## Patentansprüche

1. Ein *Corynebacterium ammoniagenes* CJIP009 mit der Zugangsnummer KCCM-10226 **gekennzeichnet durch** Akkumulieren von 5'-Inosinsäure in einer hohen Konzentration und Ausbeute **durch** direkte Fermentation und mit einer Resistenz gegen L-Glutamin-Analoge, ausgewählt aus Azaserin oder 6-Diazo-5-oxo-L-norteucin (DON), und Resistenz gegen L-Prolin-Analoge, ausgewählt aus 3,4-Dehydroprolin, L-azetidin-2-Carboxylsäure, L-Thiaziolidin-4-Carboxylsäure, (S)2,2-Dimethyl-4-oxazolid-Carboxylsäure, (S)-5,5-Dimethyl-4-Thiazolid-Carboxylsäure, (4S,2RS)-2-ethyl-4-Thiazolin-Carboxylsäure, (2S, 4S)-4-Hydroxy-2-Pyrrolin-Carboxylsäure, 2-Piperidin-Carboxylsäure oder 2,5-Pyrrolidindion.

2. Das *Corynebacterium ammoniagenes,* wie in Anspruch 1 beansprucht, **dadurch gekennzeichnet, dass** das *Corynebacterium ammoniagenes* eine Guanin- oder Xanthine-Leaky-Mutante ist oder der Mikroorganismus Adenin benötigt.

3. Das *Corynebacterium ammoniagenes,* wie in Anspruch 1 beansprucht, **dadurch gekennzeichnet, dass**, die minimale Wachstums-Inhibitions-Konzentration von Lysozym, Zellwand-Degradations-Enzym, gleich 8 µg/ml ist.

4. Das *Corynebacterium ammoniagenes,* wie in Anspruch 1 beansprucht, **dadurch gekennzeichnet, dass**, es eine Resistenz gegen Streptomycin hat, um Kontamination zu verhindern, welche während der Fermentation auftritt.

5. Ein Prozess zum Herstellen von 5'-Inosinsäure, **gekennzeichnet durch** Kultivieren des *Corynebacterium ammoniagenes* entsprechend Anspruch 1, gefolgt vom Sammeln der kultivierten Substanzen.

6. Der Prozess, wie in Anspruch 5 beansprucht, **gekennzeichnet dadurch, dass** das *Corynebacterium ammoniagenes* entsprechend Anspruch 1 kultiviert wird auf einem Zucht-Medium bei 30°C für 24 Stunden, kultiviert und aktiviert wird einem Fermentor-Kultivierungs-Medium bei 28-34°C, 900rpm und pH 7,2 für 1-2 Tage, kultiviert wird auf einem Fermentor-Haupt-Medium bei 30°C, 900rpm und pH 7,2 für 5-6 Tage und anschließend bei Vorliegen eines reduzierenden Zuckers bei 2% in der Kultivierungslösung, die ersten, zweiten, dritten und vierten zusätzlichen Zucker vermischt werden mit Fruktose, Glukose und Melassen und die entsprechenden letztendlichen Zucker hinzugefügt und kultiviert werden, so dass sie 32% ergeben.

## Revendications

1. *Corynebacterium ammoniagenes* CJIP009 ayant le numéro d'accès KCCM-10226, **caractérisé par** l'accumulation d'acide 5'-inosinique en une concentration et avec un rendement élevés au moyen d'une fermentation directe et ayant une résistance à des analogues de la L-glutamine choisis parmi l'azasérine et la 6-diazo-5-oxo-L-norleucine (DON), et une résistance à des analogues de la L-proline choisis parmi la 3,4-déshydroproline, l'acide L-azétidine-2-carboxylique, l'acide L-thiazolidine-4-carboxylique, l'acide (S)-2,2-diméthyl-4-oxazolidecarboxylique, l'acide (S)-5,5-diméthyl-4-thiazolidecarboxylique, l'acide (4S,2RS)-2-éthyl-4-thiazolinecarboxylique, l'acide (2S,4S)-4-hydroxy-2-pyrrolinecarboxylique, l'acide 2-pipéridinecarboxylique et la 2,5-pyrrolidinedione.

2. *Corynebacterium ammoniagenes* selon la revendication 1, **caractérisé en ce que** le *Corynebacterium ammoniagenes* est du type partiellement fonctionnel pour la guanine ou la xanthine ou est le micro-organisme requérant de l'adénine.

3. *Corynebacterium ammoniagenes* selon la revendication 1, **caractérisé en ce que** la concentration inhibitrice de croissance minimale de lysozyme, une enzyme de dégradation de la paroi cellulaire, est de 8 µg/ml.

4. *Corynebacterium ammoniagenes* selon la revendication 1, **caractérisé en ce qu'**il a une résistance à la streptomycine afin d'empêcher une contamination de se produire durant la fermentation.

5. Procédé pour produire de l'acide 5'-inosinique, **caractérisé par** la culture du *Corynebacterium ammoniagenes* selon la revendication 1 suivie de la collecte des substances cultivées.

6. Procédé selon la revendication 5, **caractérisé en ce que** le *Corynebacterium ammoniagenes* selon la revendication 1 est cultivé sur un milieu d'ensemencement à 30°C pendant 24 heures, cultivé et activé sur un milieu d'ensemencement en fermenteur à 28-34°C, à 900 t/min et à pH 7,2 pendant 1-2 jours, cultivé sur un milieu principal en fermenteur à 30°C, à 900 t/min et à pH 7,2 pendant 5-6 jours, et ensuite, quand un sucre réducteur est présent à raison de 2 % dans la solution de culture, les premier, deuxième, troisième et quatrième sucres additionnels sont mélangés avec du fructose, du glucose et de la mélasse et les sucres finals respectifs sont ajoutés et cultivés pour être à 32 %.
